# EUROPEAN PATENT SPECIFICATION

(11) **EP 0 649 298 B1**
(45) Date of publication and mention of the grant of the patent: **15.12.1999**
(21) Application number: 93914872.2
(22) Date of filing: 05.07.1993
(51) Int. Cl.: A61F 5/01

(54) **WRIST BRACES**
HANDGELENKSSTÜTZEN
ATTELLES POUR LE POIGNET

(30) Priority: 03.07.1992 GB 9214224
(43) Date of publication of application: 26.04.1995
(73) Proprietor: WYNDHAM, Patricia, Petersfield, Hants GU32 1DQ (GB)
(72) Inventor: CONRAN, Sebastian, London W14 0RH (GB)
(74) Representative: Butler, Michael John
(86) International application number: GB9301395
(87) International publication number: WO9401066

(56) References cited:
- EP-A- 0 162 610
- EP-A- 0 275 613
- DE-U- 8 811 364
- US-A- 1 469 315
- US-A- 3 327 703
- US-A- 4 445 507

## Description

The present invention relates primarily to wrist braces and, in particular, but not exclusively to wrist braces for rheumatoid arthritics.

Wrist braces are worn by rheumatoid arthritics to restrict movement of the wrist joint and are designed to support the wrist whilst still allowing the fingers and thumb the freedom to move. Although the brace substantially immobilizes the wrist, it is important that the fingers and thumb are still able to move freely so that the finger and thumb joints do not stiffen and the muscles weaken.

Wrist braces are not only worn by rheumatoid arthritics but may also be worn to support the wrist after injuries such as sprains and strains. Braces are also worn as orthopaedic supports, for example, after a wrist which has been broken is removed from a plaster cast. It has also been found that wrist braces are of benefit as a remedial aid to those suffering from repetitive strain injury which may occur, for example, from over use of a computer terminal. Finally wrist braces have been found to be of use by sportsmen and workmen in order to prevent injuries to the wrist occurring as a result of lifting heavy objects or weights.

One type of wrist brace which is currently widely available has a glove made of a material stretchable only in the longitudinal direct of the brace i.e. in the direction of the length of the arm. A metal support member is arranged to extend internally of the glove, from the palm, down the inside of the arm. The glove is adjusted to the individual wearer's requirements by adjustable fasteners arranged on the outside of the arm, i.e. on the side opposite to the support member. The fasteners usually take the form of a number of parallel releasable straps of e.g. Velcro (Registered Trade Mark) releasable straps of e.g. Velcro (Registered Trade Mark) which extend between opposed sides of glove material. In order to put the brace on, the straps are unfastened so that the hand can be inserted into the glove. The straps are then adjusted and fastened to accommodate the individual wearer's requirements.

However, the use of such straps has several disadvantages. The straps require a relatively large amount of force to be applied by the patient to undo the straps which is a problem for those patients with weakened joints and in particular those patients with rheumatoid arthritis in both hands. This problem is further exacerbated by the tendency of the straps to slip so that the straps must be undone and tightened several times during the day. This repeated exertion has been found actually to aggravate the problems suffered by the patient.

A further type of wrist brace, disclosed in US 3327703 (Gamm) comprises a sleeve adapted to extend from a hand region of a wearer to a forearm region, the sleeve being made from a material which is elastic in the circumferential direction, and a preformed support removably positioned in the sleeve, the support extending longitudinally of the sleeve for supporting the wrist of a wearer.

The wrist brace of the present invention is characterised in that the sleeve is also elastic in the longitudinal direction and in that the sleeve is provided with a longitudinally extending fastening means permitting the brace to be fastened about the wrist of a wearer by pulling together portions of the sleeve material against its circumferential elasticity, or permitting the brace to be removed.

Thus, by using a longitudinally extending fastening means, preferably in the form of a zip, the wearer does not have to continually adjust the fastening means as once the fastening means is closed, it will remain so until opened by the user.

Furthermore, a zip can be easily opened and closed by pulling in the longitudinal direction without the patient having to exert undue force on the zip, in an outward direction. Another advantage of using a zip or the like, is that the brace can be easily machine washed with or without the support member in place. With the known wrist braces, it has not been possible to successfully wash the brace as the straps have tended to snag on other items during the washing process.

To further facilitate the opening and closing of the zip it is preferred that the zip have a large pull of such a shape that it can be easily grasped by an arthritic.

The zip is preferably large toothed as this reduces the effort required by the user to open and close the zip.

It is preferred that the support is positioned between the fastening means and the wearer. This will reduce any irritation that might be caused by unevenness of the fastening means, the support acting as a shield.

It is further preferred that the support and the fastening means is arranged to extend along the inside of the arm of the wearer.

By arranging the fastening means to be along the inside of the wearer's arm, the need for twisting of the elbow joint is avoided. Furthermore, the appearance of the article is improved as the fastening means will not normally be visible, and there will be a reduced tendency for snagging.

The glove is preferably of a double layer of material. This may provide smooth edges for the glove. This feature not only improves the appearance of the glove, but also makes it more comfortable for the wearer.

Preferably the glove is of Lycra® or a material having similar properties. Lycra® has a number of advantages over the material used in the known wrist brace described earlier. In particular it can be easily washed and the material is not stained. It is also light and able to breathe which makes it comfortable for the wearer. Furthermore, the material has a pleasing appearance. This is advantageous in that it has been found that some patients are reluctant to wear wrist braces especially in public and at social events which can have a detrimental effect on the wrist joint of an arthritic. Thus with a pleasing appearance, the wrist brace will be more attractive to the patient and hence likely to be worn more often.

Another advantage with Lycra® is that it is a relatively non-abrasive fabric. As time progresses, arthritics skin becomes weak and sensitive and the material of known wrist braces are relatively abrasive. Materials other than Lycra having a low abrasive properties can also be used if desired.

In certain embodiments, additional support for the wrist may be provided in the form of an extra layer of material around the wrist joint. This additional layer of material may be the same as the material of the rest of the glove or may be of a different grade or type of material. For example the material may be thicker or less elastic in order to provide more support for the wrist.

In all of the above mentioned embodiments, it is preferred that the support member be arranged in a pocket which extends along at least part of the length of the glove, along the inside of the arm. The pocket is preferably shaped as to conform to the shape of the support member.

In one embodiment, the pocket of the wrist brace is substantially tubular and closed at one end. The support member is then inserted from the other end. A flap may be provided at the open end so as to close off the pocket to retain the member in position.

The support member is preferably of plastics material which has the advantage over metal in that it is lighter. Plastics also has the advantage that whilst the support is sufficiently rigid, it still retains a slight resiliency which make it more comfortable for the wearer. The support member may, for example, be of ABS and may be moulded using any suitable technique such as injection moulding, vacuum forming etc.

Furthermore, if the support member is of plastics it may be relatively easily adjusted so that the wrist is supported at the desired angle by using a hot air gun.

The support member may have a plurality of perforations arranged along its length and preferably in the region of the part of the support member which contacts the palm. These perforations allow perspiration to escape which makes it more comfortable for the wearer.

As mentioned earlier, in certain preferred embodiments, the support member may be arranged so as to lie between the arm of the wearer and the fastening means so as to act as a guard. Thus if the fastening means is in the form of a zip or the like, parts of the zip are prevented from pressing into the arm of the wearer.

The support member may have an elongate portion which extends along the inner arm of a wearer and an upper, wider portion on which the palm of the wearer rests. In other embodiments, the upper portion may be of a similar width to the elongate portion. The size of the upper portion may be selected in dependence on the degree of gripping required by the wearer. A smaller upper portion can make gripping an object easier as compared with a larger upper portion. Preferably the upper portion is cupped so as to provide a convex surface which conforms to the shape of the wearer's palm.

The elongate portion may have a concave cross-section so that it conforms to the contour of the inside arm. A widened portion may be provided on the elongate portion for additional strength, if required.

The upper portion may be angled relative to the elongate portion so as to hold the wrist at the desired angle. This angle may be in the region 30° to 45°. Thus the wrist cannot move any further forward than the angle defined by the supports but can move back, if possible. In this way, depending on the angle selected, the wrist is substantially immobilized.

Preferably, the support member is removable received in a pocket of the glove. Thus when the brace requires washing, the support member can be removed and the glove washed. It should be noted that the wrist brace can also be successfully washed with the support member in place, as discussed earlier.

A wrist brace according to the invention will now be described with reference to the accompanying drawings in which:
Figure 1 shows a perspective view of a wrist brace as worn;
Figure 2 shows a perspective view of a support member of Figure 1;
Figure 3 shows a side view of the support member of Figure 2;
Figure 4 shows a plan view of the support member of Figure 2; and
Figure 5 shows a perspective view of an alternate support member for use in the wrist brace of Figure 1.

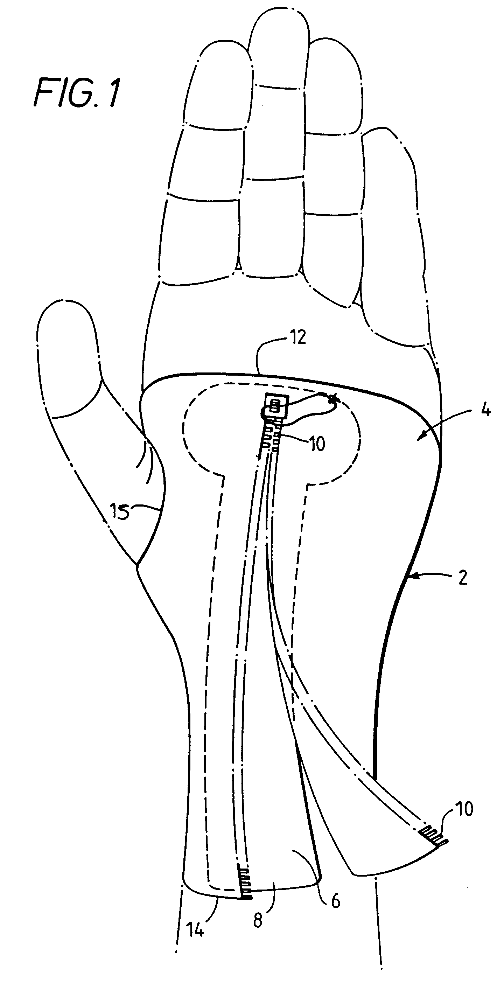

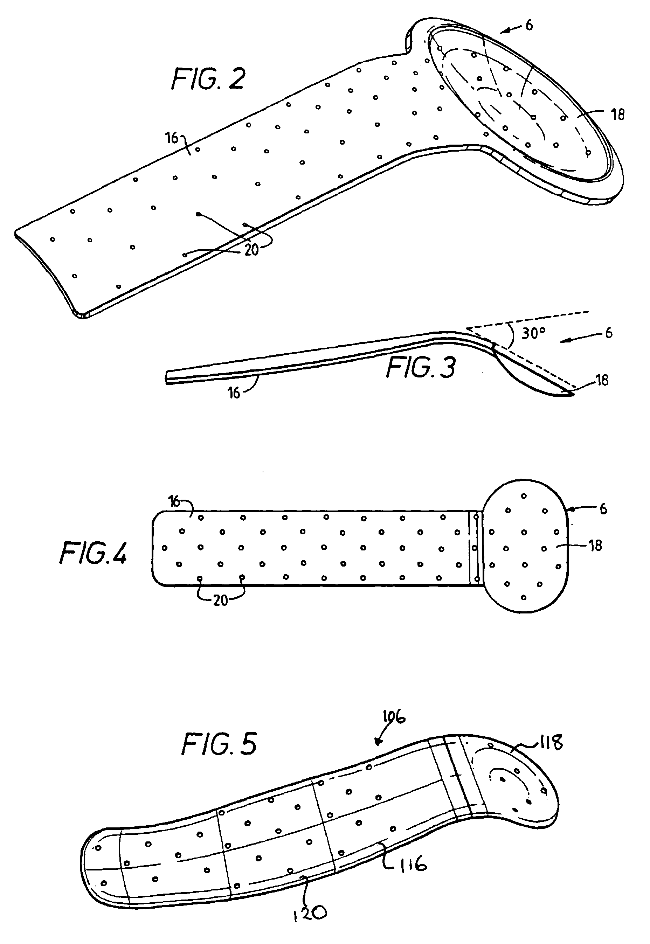

Referring first to Figure 1, the wrist brace 2 comprises a glove or sleeve 4 inside of which is arranged a support member 6 (which can be seen more clearly in Figures 2 to 4). The support member 6 is arranged in a tubular pocket 8 which extends, internally along the length of the glove 4. Arranged above the support member 6 is a zip fastener 10 which extends along much of the length of the glove.

The various components of the wrist brace will now be described in more detail. The glove 4 is of a generally tubular shape and has openings 12 and 14 at opposed ends of the glove 4. At one end of the glove 4 is the opening 12 through which the fingers and upper part of the palm extend. The fingers of the wearer are thus able to move freely. The arm of the wearer extends through the other opening 14 of the glove 4. The glove 4 has an additional opening 15, adjacent the opening 12 for the fingers, for the thumb of the wearer. The glove 4 is made from a double layer of Lycra® or any other suitable material, and is stretchable in two perpendicular directions, ie. along the glove and circumferentially of it.

As can be seen from Figures 2 to 4, the support member 6 has an elongate portion 16 and a cupped portion 18. The cupped portion 18 is shaped so as to conform generally to the shape of the part of the palm which rests against the cupped portion 18. The cupped portion 18 extends at approximately 30° to the longitudinal axis of the elongate portion 16 so that the wrist is prevented from being bent at an angle less than 30° relative to the extended position of the wrist. The elongate portion 16 is arranged so as to extend along the length of the arm and has, in the direction perpendicular to the length of the arm, a slightly concave cross-section so as to conform to the contour of the arm.

The support member 6 is made of a substantially rigid plastics material such as ABS but has a degree of resiliency. The support member has a number of through holes 20 through which the perspiration of the wearer can escape. The angle between the cupped portion and the elongate portion can be adjusted for a user by e.g. heating the plastic with a hot air gun, and bending it.

The support member 6 is arranged inside the glove 4 in tubular pocket 8. Pocket 8 is open at the end closest to the fingers and the support member 6 is inserted into the pocket 8, the other end of which is closed. The open end of the pocket has a flap (not shown) which is tucked over the open end of the pocket 8 to retain the support member 6 in place.

The pocket 8 and support member 6 are arranged so as to be below the zip 10 to provide protection to the user from any part of the zip which might otherwise press into the arm of the wearer. The zip 10 itself is of the type which has a large teeth to facilitate opening and closing. The zip also has a loop through which the fingers of the wearer can be inserted for opening and closing the zip 10.

Figure 5 shows an alternate support member 106 which can be used in the wrist brace of Figure 1. This alternate support member 106 is similar to that described earlier. However, support member 106 has a smaller cupped portion 118 which has a width similar to that of the elongate portion 116. If the cupped portion is too large the wearer can have difficulty in grasping objects. Accordingly, in some embodiments, such as shown in Figure 5, the cupped portion is smaller. The elongate portion 116 has a widened portion 120 to increase the strength of the support member 106.

The following procedure is carried out in order to put on the brace having either of the support members illustrated. The zip 10 is moved to its opened position and the hand is inserted into the upper end of the glove 4 so that the fingers and thumb project from their respective openings 12, 14 and 15. The zip is then closed by pulling loop downwardly. Since Lycra is stretchable in more than one direction, the brace is adjusted automatically to the size of the wearer. The brace needs no further adjustment until it has to be taken off when the above steps are carried out in reverse.

## Claims

1. A wrist brace (2) comprising a sleeve (4) adapted to extend from a hand region of a wearer to a forearm region, the sleeve being made from a material which is elastic in the circumferential direction, and a pre-formed support (6) removably positioned in the sleeve, the support extending longitudinally of the sleeve for supporting the wrist of a wearer, characterised in that the sleeve (4) is also elastic in the longitudinal direction and in that the sleeve (4) is provided with a longitudinally extending fastening means (10) permitting the brace to be fastened about the wrist of a wearer by pulling together portions of the sleeve material against its circumferential elasticity, or permitting the brace to be removed.

2. A wrist brace as claimed in claim 1, characterised in that the fastening means (10) is a zip fastener.

3. A wrist brace as claimed in claim 2 characterised in that the zip fastener (10) has a large, easy to grip, pull.

4. A wrist brace as claimed in claim 1, 2 or 3 characterised in that the sleeve (4) is in the form of a glove having a wrist opening (14) at one end, an opening (12) for the fingers and an opening (15) for the thumb at the other end, and a longitudinal opening extending from the wrist opening (14) part way towards the fingers opening (12), the longitudinal opening being provided with the fastening means (10).

5. A wrist brace as claimed in any preceding claim, characterised in that the support (6) is positioned between the fastening means (10) and the wearer.

6. A wrist brace as claimed in claim 5, characterised in that the support (6) and fastening means (10) extend along the inside of the arm of the wearer.

7. A wrist brace as claimed in claim 6, characterised in that the support (6) comprises an elongate portion (16) and a cupped portion (18) which provides a convex surface arranged to conform to the shape of a wearer's palm.

8. A wrist brace as claimed in claim 7 characterised in that the elongate portion (16) of the support has a concave cross section which is arranged to conform to the insde arm of a wearer.

9. A wrist brace as claimed in claim 6, 7 or 8, characterised in that the cupped-portion (18) is angled relative to the elongate portion (16) at an angle in the range of 30 to 45 degrees.

10. A wrist brace as claimed in any preceding claim, characterised in that the support (6) is perforated.

11. A wrist brace as claimed in any preceding claim, characterised in that the support (6) is mounted within a pocket (8) of the sleeve (4).

12. A wrist brace as claimed in any preceding claim characterised in that the support (6) is of plastics material and is sufficiently rigid to support the wrist whilst having a degree of resiliency.

13. A wrist brace as claimed in any preceding claim, characterised in that the sleeve (4) has a layer of additional material to provide additional support of the wrist.

## Patentansprüche

1. Handgelenkstütze mit einer Manschette (4), welche derart ausgebildet ist, daß sie sich von einem Handbereich eines Trägers zu einem Unterarmbereich erstreckt, wobei die Manschette aus einem in Umfangsrichtung elastischen Material hergestellt ist, und einer entfernbar in der Manschette positionierten vorgeformten Unterstützung(6), wobei die Unterstützung sich zum Unterstützen des Handgelenks eines Trägers in der Manschette in Längsrichtung erstreckt,
**dadurch gekennzeichnet**,
daß die Manschette (4) auch in Längsrichtung elastisch ist, und daß die Manschette (4) mit sich in Längsrichtung erstreckenden Befestigungsmitteln (10) ausgebildet ist, welche eine Befestigung der Stütze um das Handgelenk eines Trägers durch Zusammenziehen von Abschnitten des Manschettenmaterials gegen seine umfängliche Elastizität, oder eine Entfernung der Stütze erlauben.

2. Handgelenkstütze nach Anspruch 1, dadurch gekennzeichnet, daß die Befestigungsmittel (10) als Reißverschluß ausgebildet sind.

3. Handgelenkstütze nach Anspruch 2, dadurch gekennzeichnet, daß der Reißverschluß (10) einen großen, in einfacher Weise greifbaren Zug aufweist.

4. Handgelenkstütze nach einem der Ansprüche 1, 2 oder 3, dadurch gekennzeichnet, daß die Manschette (4) in Form eines Handschuhs ausgebildet ist, welcher eine Handgelenksöffnung (14) an einem Ende und eine Öffnung (12) für die Finger und eine Öffnung (15) für den Daumen an dem anderen Ende, sowie eine Längsöffnung, die sich von der Handgelenksöffnung (14) einen Teil der Strecke in Richtung der Fingeröffnung (12) erstreckt, aufweist, wobei die Längsöffnung mit den Befestigungsmitteln (10) ausgebildet ist.

5. Handgelenkstütze nach einem der vorstehenden Ansprüche, dadurch gekennzeichnet, daß die Unterstützung (6) zwischen den Befestigungsmitteln (10) und dem Träger angeordnet ist.

6. Handgelenkstütze nach Anspruch 5, dadurch gekennzeichnet, daß die Unterstützung (6) und die Befestigungsmittel (10) sich entlang der Innenseite des Armes des Trägers erstrecken.

7. Handgelenkstütze nach Anspruch 6, dadurch gekennzeichnet, daß die Unterstützung (6) einen länglichen Abschnitt (16) und einen schalenförmigen Abschnitt (18), welcher eine konvexe Oberfläche, die zur Anpassung an die Form einer Handfläche eines Trägers eingerichtet ist, aufweist.

8. Handgelenkstütze nach Anspruch 7, dadurch gekennzeichnet, daß der längliche Abschnitt (16) der Unterstützung einen konkaven Querschnitt aufweist, welcher zur Anpassung an den Innenarm eines Trägers ausgebildet ist.

9. Handgelenkstütze nach einem der Ansprüche 6, 7 oder 8, dadurch gekennzeichnet, daß der schalenförmige Abschnitt (18) bezüglich des länglichen Abschnitts (16) einen Winkel im Bereich von 30° bis 45° bildet.

10. Handgelenkstütze nach einem der vorstehenden Ansprüche, dadurch gekennzeichnet, daß die Unterstützung (6) perforiert ausgebildet ist.

11. Handgelenkstütze nach einem der vorstehenden Ansprüche, dadurch gekennzeichnet, daß die Unterstützung (6) innerhalb einer Tasche(8) der Manschette (4) angebracht ist.

12. Handgelenkstütze nach einem der vorstehenden Ansprüche, dadurch gekennzeichnet, daß die Unterstützung (6) aus einem Kunststoffmaterial hergestellt ist und eine ausreichende Stärke bzw. Festigkeit zum Unterstützen des Handgelenks und gleichzeitig ein Maß an Rückfederung aufweist.

13. Handgelenkstütze nach einem der vorstehenden Ansprüche, dadurch gekennzeichnet, daß die Manschette (4) eine Schicht eines zusätzlichen Materials zur Schaffung einer zusätzlichen Unterstützung des Handgelenks aufweist.

## Revendications

1. Attelle de poignet (2) comprenant un manchon (4) adapté pour s'étendre d'une région de la main d'un porteur à une région de l'avant-bras, le manchon étant en une matière qui est élastique dans la direction périphérique, et un support préformé (6) positionné de façon amovible dans le manchon, le support s'étendant le long du manchon pour supporter le poignet d'un porteur, caractérisée en ce que le manchon (4) est également élastique dans la direction longitudinale et en ce que le manchon (4) est pourvu d'un moyen de fixation (10) s'étendant longitudinalement qui permet la fixation de l'attelle autour du poignet d'un porteur en tirant ensemble des portions de la matière de manchon à l'encontre de son élasticité périphérique, ou qui permet le retrait de l'attelle.

2. Attelle de poignet selon la revendication 1, caractérisée en ce que le moyen de fixation (10) est une attache à fermeture éclair.

3. Attelle de poignet selon la revendication 2, caractérisée en ce que l'attache à fermeture éclair (10) comporte une grosse tirette facile à saisir.

4. Attelle de poignet selon la revendication 1, 2 ou 3, caractérisée en ce que le manchon (4) se présente sous la forme d'un gant comportant une ouverture de poignet (14) à une extrémité, une ouverture (12) pour les doigts et une ouverture (15) pour le pouce à l'autre extrémité, et une ouverture longitudinale s'étendant de l'ouverture de poignet (14) en partie en direction de l'ouverture (12) pour les doigts, l'ouverture longitudinale étant dotée du moyen de fixation (10).

5. Attelle de poignet selon l'une quelconque des revendications précédentes, caractérisée en ce que le support (6) est positionné entre le moyen de fixation (10) et le porteur.

6. Attelle de poignet selon la revendication 5, caractérisée en ce que le support (6) et le moyen de fixation (10) s'étendent le long de la partie intérieure du bras du porteur.

7. Attelle de poignet selon la revendication 6, caractérisée en ce que le support (6) comprend une portion étendue (16) et une portion en cuvette (18) qui présente une surface convexe agencée pour se conformer à la forme de la paume d'un porteur.

8. Attelle de poignet selon la revendication 7, caractérisée en ce que la portion étendue (16) du support a une forme concave en coupe transversale qui est agencée pour se conformer à la partie intérieure du bras d'un porteur.

9. Attelle de poignet selon la revendication 6, 7 ou 8, caractérisée en ce que la portion emboutie (18) fait un angle par rapport à la portion étendue (16) dans la gamme de 30 à 45 degrés.

10. Attelle de poignet selon l'une quelconque des revendications précédentes, caractérisée en ce que le support (6) est perforé.

11. Attelle de poignet selon l'une quelconque des revendications précédentes, caractérisée en ce que le support (6) est monté à l'intérieur d'une poche (8) du manchon (4).

12. Attelle de poignet selon l'une quelconque des revendications précédentes, caractérisée en ce que le support (6) est en matière plastique et est suffisamment rigide pour supporter le poignet tout en ayant un certain degré d'élasticité.

13. Attelle de poignet selon l'une quelconque des revendications précédentes, caractérisée en ce que le manchon (4) comporte une couche de matière additionnelle pour réaliser un support additionnel du poignet.
